# EUROPEAN PATENT SPECIFICATION

(11) **EP 1 550 439 B1**
(45) Date of publication and mention of the grant of the patent: **08.03.2006**
(21) Application number: 03029897.0
(22) Date of filing: 29.12.2003
(51) Int. Cl.: A61K 9/16, A61K 9/20, A61K 38/11

(54) **Method for preparing a solid dosage form of desmopressin**
Verfahren zur Herstellung einer festen oralen Dosierungsform von Desmopressin
Procédé pour la préparation d'une forme solide de dosage de Desmopressine

(43) Date of publication of application: 06.07.2005
(73) Proprietor: Ferring B.V., 2132 JX Hoofddorp (NL)
(72) Inventor: Wannerberger, Kristin, 223 51 Lund (SE); Lindner, Hans, Dr., 1828 Frederiksberg (DK); Olsson, Lars-Erik, 215 67 Malmö (SE); Svensson, Ann Elisabeth, 234 37 Lomma (SE)
(74) Representative: HOFFMANN EITLE

(56) References cited:
- WO-A-95/18602
- WO-A-03/094886
- US-A1- 2003 091 637

## Description

### Field of the Invention

The present invention relates to a novel method for the preparation of a solid dosage form of desmopressin, or a pharmaceutically acceptable salt thereof.

### Background

Desmopressin, also known as dDAVP, is a nonapeptide and the therapeutically active ingredient (as the acetate salt) in the pharmaceutical product Minirin®, which is marketed *inter alia* as a nasal spray and a tablet formulation. Desmopressin is primarily used in the treatment of primary nocturnal enuresis, *i.e.* bedwetting, in children, but it is approved also for the treatment of nocturia and diabetes insipidus. The first market introduction of the tablet formulation was in Sweden in 1987. The composition of the marketed tablet form of desmopressin has remained the same to date.

The tablet form of desmopressin was first disclosed as set forth in the patent US 5,047,398. The subsequently issued marketing authorisations relate to a tablet where *i.a.* the mannitol, talc and cellulose components exemplified in US 5,047,398 are replaced with potato starch. In addition to desmopressin acetate and potato starch, the present tablet components are lactose, polyvinylpyrrolidone (PVP) and magnesium stearate that together form a homogeneous tablet compressed from a granulate. This composition is *inter alia* disclosed in the publication WO 03/94886 A1 (see page 28).

Since desmopressin is a nonapeptide containing a disulfide bond, its stability must always be considered. Representative publications addressing the problem of the stability of desmopressin in pharmaceutical formulations are EP 1255557 A1, EP 752877 A1 and EP 710122 A1.

Desmopressin containing granulate has to date been prepared in a wet granulation process involving a sequence of several sieving and mixing steps performed at ambient temperature and humidity followed by drying (*cf*. example 1 herein). One of the objectives of that procedure is to keep shearing forces that desmopressin may be subjected to at a minimum level. The main disadvantages of said procedure is that it is rather time-consuming and labor intensive.

The publication WO 97/15297 A1 (examples 6 and 10) discloses a wet granulation method for the preparation of a buccal delivery system for desmopressin.

As a mixture of water and ethanol is used as the granulation liquid in the prior art granulate preparation, the resulting tablet inevitably contains solvent residues, typically 5-6% of water and 0.1% of ethanol (percentage by weight) Complete removal of solvent residues by drying is impractical, as conditions for complete drying of solid dosage forms tend to be either too costly in industrial scale or potentially thermally damaging to the desmopressin. The primary purpose of the added ethanol is to shorten the time of drying (via an azeotrope).

US 2003/0091637 A1 discloses the use of a specific copolymer as the coating agent for a pharmaceutical core. The pharmaceutical active principle can be, for example, a peptide hormone. The coating may be conducted using a fluidised bed apparatus.

WO 03/094886 discloses the preparation of tablets containing desmopressin using a conventional wet granulation process.

It is an objective of the present invention to overcome the aforementioned disadvantages.

### Disclosure of the Invention

The present invention relates to a method for the preparation of a solid dosage form of desmopressin, or a pharmaceutically acceptable salt thereof, comprising granulating desmopressin or a pharmaceutically acceptable salt thereof and at least one excipient, carrier or diluent or mixture thereof in a fluid bed granulation apparatus, wherein the resulting desmopressin containing granulate is suitable for compression to a pharmaceutically acceptable tablet. More specifically, said processing comprises providing conditions to provide mixing and shearing action. Said granulation typically comprises adjusting fluidising air flow and processing temperature and time.

Standard literature (see *"Pharmaceutical Dosage Forms; Tablets",* vol.1, pages 297-298, Eds. H.A. Lieberman, L. Lachman and J.B. Schwartz, Marcel Dekker, Inc., New York and Basel, 1989) teaches that the conditions involved in fluid bed granulation may be harmful *e.g*. to enzymes. More specifically, the heat and moisture combined with the circulating air and particles in a fluid bed granulation process generate significant shearing and abrasion forces with the purpose of providing a granulate having flow properties ideal for tablet compression at industrial scale and speed. Such flow properties are due to the resulting smooth surface structure of the granulate subjected to said shearing and abrasion forces.

It is a surprising observation that a molecule as sensitive as desmopressin can withstand the processing conditions of fluid bed granulation. The most significant advantages of the method of the present invention are the short processing time compared to conventional wet granulation, and the excellent flow properties for compression of the resulting granulate.

Fluid bed granulation *per se* is a conventional technology, and it is extensively disclosed in various standard literature, such as *"Pharmaceutical Dosage Forms; Tablets",* vol.3, pages 27-29, Eds. H.A. Lieberman, L. Lachman and J.B. Schwartz, Marcel Dekker, Inc., New York and Basel, 1990) and *"Pharmaceutics - The science of dosage form design",* pages 625-627; Ed. M.E. Aulton, Churchill Livingstone, Edinburgh, London, Melbourne and New York 1988. The proper selection of the general equipment set up & processing conditions is therefore within the capacity of a person skilled in the art of manufacturing pharmaceutical formulations. Examples of commercial providers of apparatus adapted for fluid bed granulation are Aeromatic-Fielder AG, CH (Strea series) and Glatt GmbH, DE.

In a preferred embodiment of the present method, said desmopressin containing granulate is prepared by a process comprising the steps of:
i) providing a powder comprising, or consisting of, at least one excipient, carrier or diluent, or mixture thereof;
ii) providing a granulation liquid containing a solvent and desmopressin, or a pharmaceutically acceptable salt thereof, and optionally a binder; and
iii) contacting said granulation liquid, preferably by spraying, with said powder within said apparatus, wherein the fluidising air flow and processing temperature and time are simultaneously, and optionally also after said contacting is completed, adjusted to provide said mixing and shearing action.

The processing conditions of fluid bed granulation usually also provide drying while the fluidisation is ongoing, *i.e.* also during said step iii). Continued processing conditions after said contacting thus provide further drying in addition to the mixing and shearing action. As an example, a spraying operation in fluid bed granulation is typically performed at a constant spraying rate over a time period of from 10 to 60 minutes. Optionally, the spraying is followed by continued processing conditions for 10 to 240 minutes if further drying, mixing and/or shearing action is desired.

Said solvent is preferably water, which is a particularly advantageous aspect of the present invention. It is noteworthy that the use of water as sole solvent nevertheless keeps the time of drying short, whereas explosion risks and organic solvent exposure are reduced while providing a granulate of required quality. In addition, the granulation process is simplified by removing a component.

Fluidising air flow refers to an air flow that is sufficient to accomplish fluidisation of the powder and resulting granulate within the fluid bed granulation apparatus. The required air flow depends upon several parameters, including particle size and density. As a nonlimiting example, the air flow may be in the range of from 10 to 2 500 m³/h, preferably from 20 to 1 500 m³/h. Different operating scales will inherently require somewhat different fluidising air flows. Selecting an optimal flow for the operating scale in question is not an impractical burden for a person skilled in the art, as the machinery *per se* required in the practising of the present invention is commercially available and thus of a conventional nature.

Said processing temperature is typically in the range of from 25 to 80°C, preferably from 30 to 60°C. Temperature ranges of from 35 to 55°C and from 40 to 50°C are also conceivable.

It is preferred that said processing time is in the range of from 10 to 240 minutes. For practical purposes, the process is typically regarded as complete when the formed granulate, which is also dried during the process, reaches a water content that is essentially equal to that of said powder comprising excipient, carrier or diluent.

In many cases the terms excipient, diluent and carrier can be used interchangeably, and they may even refer to one and the same substance, or to a mixture of similar such substances. The proper use and understanding of these terms is well known to a person skilled in the art.

In the present method it is preferred that said excipient, carrier or diluent is selected from cellulose, starch and lactose. As used herein, the term cellulose includes, taken alone or in mixture, neat cellulose, microcrystalline cellulose, carboxymethyl cellulose, hydroxypropyl methylcellulose as well as other variants thereof that may be employed in pharmaceutical formulations. As used herein, the term starch includes, taken alone or in mixture, potato starch, wheat starch, corn starch, rice starch and sheared and/or acid-hydrolysed variants of the aforementioned starches as well as other variants of starch that are typical in pharmaceutical formulations. The lactose type used is preferably lactose-α-monohydrate.

As indicated above the present solid dosage form may optionally comprise at least one further additive typically selected from a disintegrating agent, binder, lubricant, flavoring agent, preservative, colorant and any suitable mixture thereof. Examples of additives that may be considered in practising the present invention are found in *"Handbook of Pharmaceutical Excipients";* Ed. A.H. Kibbe, 3^{rd} Ed., American Pharmaceutical Association, USA and Pharmaceutical Press UK, 2000.

In a preferred embodiment of the present method, said desmopressin containing granulate is compressed to a tablet, preferably in a process where a lubricant is added to said granulate before compression thereof.

Said lubricant is typically selected from a group consisting of stearic acid, salts or esters of stearic acid, hydrogenated vegetable oils, magnesium oxide, polyethylene glycol, sodium lauryl sulphate and talc, and mixtures thereof. Preferably said lubricant is selected from magnesium stearate, calcium stearate, zinc stearate, glyceryl palmitostearate and sodium stearyl fumarate, and mixtures thereof. Magnesium stearate is most preferred. The content of lubricant is typically from 0.05 to 1.0, preferably from 0.25 to 0.50, percent by weight of each unit of solid dosage form.

The practising of the present method preferably includes a binder, e.g. PVP. Typically an amount of binder of from 1 to 6 percent by weight of each unit of solid dosage form is employed.

In the most preferred embodiment said solid dosage form lacks an enteric coating. By avoiding an enteric coating the preparation of the solid dosage form of the present invention is further simplified.

The solid dosage form as eventually prepared preferably lacks an agent that exerts buffering capacity at a pH of from 2 to 6.

The method of the present invention most preferably provides an amount of desmopressin acetate of from 20 to 600 µg per unit of said solid dosage form.

Said solid dosage form is preferably selected from a group consisting of tablets, granulate powder, lozenge, cachet, and wafer sheet. A tablet is most preferred.

The present pharmaceutical composition in a solid dosage form is typically a perorally available tablet. A tablet may be manufactured by compression of a granulate by procedures well established in the art. Examples of suitable tablet compressing equipment are rotary presses provided by Elizabeth-Hata International, USA, and Courtoy NV, BE. For a comprehensive overview of pharmaceutical tablet manufacturing, see "Tabletting" (by N.A. Armstrong) in the aforementioned *"Pharmaceutics - The science of dosage form design",* pages 647-668.

The following illustrates the present invention in more detail.

### Experimental

### Comparative Example 1 (prior art): Preparation of a tablet containing desmopressin acetate via wet granulation

Lactose (900 g, Pharmatose 150M; provided by DMV, NL) and potato starch (550 g, AmylSolVät; provided by Lyckeby Stärkelse AB, SE) are mixed in a planetary mixer for 15 minutes at room temperature and sieved through a 1 mm sieve. A granulation liquid consisting of water (75 ml) and PVP (13.8 g, Kollidon® 25; provided by BASF GmbH, DE) is prepared, to which desmopressin acetate (0.75 g; provided by PolyPeptide Laboratories AB, SE) and ethanol (225 g) are added. The granulation liquid is then gradually added to the lactose/starch mixture during mixing for 20 minutes, followed by further mixing for 10 minutes at room temperature. After sieving (1.4 mm), drying for about 20 hours at 40°C and further sieving (1.4 mm), the obtained granulate is admixed with magnesium stearate (11.3 g, 1.0 mm sieved; provided by Peter Greven NV, NL) and subsequently compressed to 7500 tablets using a single punch tablet compression machine (Fette Exacta 1). A typical prepared tablet for commercial use contains 0.1 mg of desmopressin acetate and is white, convex and oval (6.8 x 9.6 mm) with a thickness of 3-4 mm and a target weight of 192 mg. It has a smooth surface without scratches or chipped edges, and shows no tendencies to lamination (so-called capping).

### Example 2: Preparation of a tablet containing desmopressin acetate via fluid bed granulation

Lactose (476.6 g, Granulac 140; provided by Meggle AG, DE) and potato starch (294.6 g, M14; provided by KMC, DK) are fed to a fluid bed granulation apparatus (Strea 1; provided by Aeromatic Fielder AG, DE) and mixed for 2 minutes in an upwards directed fluidising air flow of 25 m³/h at a set temperature of 45°C. A granulation liquid is prepared by dissolving PVP (24 g, Povidone; provided by BASF, DE) and desmopressin acetate (0.80 g; provided by PolyPeptide Laboratories AB, SE) in water (80 g). The granulation liquid is then sprayed downwards at a constant rate during 15 minutes onto the lactose/starch mixture while the latter is simultaneously subjected to an upwards directed fluidising air flow of 25 m³/h at a temperature of 45°C. When all the granulation liquid is added the same air flow and temperature is maintained for a further 20 minutes. The obtained dry granulate is then sieved (1.0 mm) and mixed with powdered magnesium stearate (4 g, 1.0 mm sieved; provided by Peter Greven NV, NL) for 2 minutes in a conventional mixer (AR400E; provided by EWREKA GmbH, DE), and subsequently compressed to 4000 tablets in a rotary punch (ø 8 mm) compression machine (Korsch XL 100; provided by Korsch, DE) with a target weight of 200 mg. Tablets with a hardness of 5 kp (1 kp = 9.81 N) and each containing 0.2 mg of desmopressin acetate were prepared in this manner. The tablets had a smooth surface without scratches or chipped edges, and no capping was observed.

## Claims

1. A method for the preparation of a solid dosage form of desmopressin, or a pharmaceutically acceptable salt thereof, comprising granulating desmopressin or a pharmaceutically acceptable salt thereof and at least one excipient, carrier or diluent or mixture thereof in a fluid bed granulation apparatus, wherein the resulting desmopressin containing granulate is suitable for compression to a pharmaceutically acceptable tablet.

2. The method according to claim 1, wherein a granulation liquid containing water as sole solvent is utilised.

3. The method according to claim 1, wherein the preparation of said granulate comprises adjusting fluidising air flow and processing temperature and time.

4. The method according to claim 3, wherein said desmopressin containing granulate is prepared by a process comprising the steps of:
i) providing a powder comprising at least one excipient, carrier or diluent, or mixture thereof;
ii) providing a granulation liquid containing a solvent and desmopressin, or a pharmaceutically acceptable salt thereof, and optionally a binder; and
iii) contacting said granulation liquid with said powder within said apparatus, wherein the fluidising air flow and processing temperature and time are simultaneously adjusted to provide mixing and shearing action.

5. The method according to claim 4, wherein said solvent is water.

6. The method according to any one of claims 3 - 5, wherein said fluidising air flow is in the range of from 10 to 2500 m³/h, preferably from 20 to 1500 m³/h.

7. The method according to any one of claims 3 - 6, wherein said processing temperature is in the range of from 25 to 80 °C, preferably from 30 to 60 °C.

8. The method according to any one of claims 3 - 7, wherein said processing time is in the range of from 10 to 240 minutes.

9. The method according to any one of claims 1 and 4 - 8, wherein said excipient, carrier or diluent is selected from cellulose, starch and lactose.

10. The method according to any one of claims 1 - 9, wherein said desmopressin containing granulate is compressed to a tablet, preferably in a process where a lubricant is added to said granulate before compression thereof.

11. The method according to claim 10, wherein said lubricant is magnesium stearate.

12. The method according to any one of claims 4 - 11, wherein a binder, preferably PVP, is present.

13. The method according to any one of claims 1 - 12, wherein said solid dosage form lacks enteric coating.

14. The method according to any one of claims 1 - 13, where said solid dosage form lacks an agent that exerts buffering capacity at a pH of from 2 to 6.

15. The method according to any one of claims 1 - 14, wherein the solid dosage form contains desmopressin acetate in an amount of from 20 to 600 µg per unit of said solid dosage form.

## Patentansprüche

1. Verfahren zur Herstellung einer festen Arzneiform von Desmopressin oder einem pharmazeutisch akzeptablen Salz davon, umfassend das Granulieren von Desmopressin oder einem pharmazeutisch akzeptablen Salz davon und wenigstens einem Exzipienten, Träger oder Verdünnungsmittel oder einer Mischung daraus in einer Fließbett-Granulierungsvorrichtung, worin das resultierende Desmopressin-haltige Granulat geeignet zur Verpressung zu einer pharmazeutisch akzeptablen Tablette ist.

2. Verfahren gemäß Anspruch 1, worin eine Granulierungsflüssigkeit, die Wasser als einziges Lösungsmittel enthält, verwendet wird.

3. Verfahren gemäß Anspruch 1, worin die Herstellung des Granulats das Einstellen von Fluidisierungsluftstrom und Verarbeitungstemperatur und -zeit umfaßt.

4. Verfahren gemäß Anspruch 3, worin das Desmopressin-haltige Granulat durch ein Verfahren hergestellt wird, das die folgenden Schritte umfaßt:
i) Bereitstellen eines Pulvers, das wenigstens einen/ein Exzipienten, Träger oder Verdünnungsmittel oder eine Mischung darauf umfaßt;
ii) Bereitstellen einer Granulierungsflüssigkeit, die ein Lösungsmittel und Desmopressin oder ein pharmazeutisch akzeptables Salz davon und gegebenenfalls ein Bindemittel enthält; und
iii) Inkontaktbringen der Granulierungsflüssigkeit mit dem Pulver innerhalb der Vorrichtung, worin der Fluidisierungsluftstrom und die Verarbeitungstemperatur und -zeit gleichzeitig eingestellt werden, um eine Misch- und Scherwirkung bereitzustellen.

5. Verfahren gemäß Anspruch 4, worin das Lösungsmittel Wasser ist.

6. Verfahren gemäß einem der Ansprüche 3 bis 5, worin der Fluidisierungsluftstrom im Bereich von 10 bis 2.500 m³/h ist, bevorzugt von 20 bis 1.500 m³/h.

7. Verfahren gemäß einem der Ansprüche 3 bis 6, worin die Verarbeitungstemperatur im Bereich von 25 bis 80°C ist, bevorzugt von 30 bis 60°C.

8. Verfahren gemäß einem der Ansprüche 3 bis 7, worin die Verarbeitungszeit im Bereich von 10 bis 240 Minuten ist.

9. Verfahren gemäß einem der Ansprüche 1 und 4 bis 8, worin der Exzipient, Träger oder das Verdünnungsmittel aus Cellulose, Stärke und Lactose ausgewählt ist.

10. Verfahren gemäß einem der Ansprüche 1 bis 9, worin das Desmopressin-haltige Granulat zu einer Tablette verpreßt wird, bevorzugt in einem Verfahren, in dem ein Schmiermittel zum Granulat vor dessen Verpressung gegeben wird.

11. Verfahren gemäß Anspruch 10, worin das Schmiermittel Magnesiumstearat ist.

12. Verfahren gemäß einem der Ansprüche 4 bis 11, worin ein Bindemittel, bevorzugt PVP, zugegen ist.

13. Verfahren gemäß einem der Ansprüche 1 bis 12, worin der festen Arzneiform ein magensaftresistenter Überzug fehlt.

14. Verfahren gemäß einem der Ansprüche 1 bis 13, worin der festen Arzneiform ein Mittel fehlt, das eine Pufferkapazität bei einem pH von 2 bis 6 ausübt.

15. Verfahren gemäß einem der Ansprüche 1 bis 14, worin die feste Arzneiform Desmopressinacetat in einer Menge von 20 bis 600 µg pro Einheit der festen Arzneiform enthält.

## Revendications

1. Procédé pour la préparation d'une forme pharmaceutique solide de la desmopressine ou d'un sel pharmaceutiquement acceptable de celle-ci, comprenant la granulation de la desmopressine ou d'un sel pharmaceutiquement acceptable de celle-ci et d'au moins un excipient, véhicule ou diluant ou un mélange de ceux-ci dans un appareil de granulation en lit fluidisé, dans lequel le granulé résultant comprenant de la desmopressine est approprié pour la compression en un comprimé pharmaceutiquement acceptable.

2. Procédé selon la revendication 1, dans lequel un liquide de granulation contenant de l'eau en tant qu'unique solvant est utilisé.

3. Procédé selon la revendication 1, dans lequel la préparation desdits granulés comprend le réglage du débit d'air de fluidisation et de la température et du temps de traitement.

4. Procédé selon la revendication 3, dans lequel ledit granulé comprenant de la desmopressine est préparé par un procédé comprenant les étapes de :
i) fourniture d'une poudre comprenant au moins un excipient, véhicule ou diluant ou un mélange de ceux-ci ;
ii) fourniture d'un liquide de granulation contenant un solvant et la desmopressine ou un sel pharmaceutiquement acceptable de celle-ci et facultativement un liant ; et
iii) la mise en contact dudit liquide de granulation avec ladite poudre dans ledit appareil, dans lequel le débit d'air de fluidisation et la température et le temps de traitement sont simultanément réglés pour fournir une action de mélange et de cisaillement.

5. Procédé selon la revendication 4, dans lequel ledit solvant est l'eau.

6. Procédé selon l'une quelconque des revendications 3 à 5, dans lequel le débit d'air de fluidisation se situe dans la fourchette entre 10 et 2500 m³/h, de préférence entre 20 et 1500 m³/h.

7. Procédé selon l'une quelconque des revendications 3 à 6, dans lequel ladite température de traitement se situe dans la fourchette entre 25 et 80°C, de préférence entre 30 et 60°C.

8. Procédé selon l'une quelconque des revendications 3 à 7, dans lequel ledit, temps de traitement se situe dans la fourchette entre 10 et 240 minutes.

9. Procédé selon l'une quelconque des revendications 1 et 4 à 8, dans lequel ledit excipient, véhicule ou diluant est choisi parmi la cellulose, l'amidon et le lactose.

10. Procédé selon l'une quelconque des revendications 1 à 9, dans lequel ledit granulé sous forme de desmopressine est compressé en un comprimé, de préférence selon un procédé où le lubrifiant est ajouté auxdits granulés avant la compression de ceux-ci.

11. Procédé selon la revendication 10, dans lequel ledit lubrifiant est le stéarate de magnésium.

12. Procédé selon l'une quelconque des revendications 4 à 11, dans lequel un liant, de préférence la P.V.P., est présent.

13. Procédé selon l'une quelconque des revendications 1 à 12, dans lequel la forme pharmaceutique solide manque d'enrobage gastrorésistant.

14. Procédé selon l'une quelconque des revendications 1 à 13, dans lequel ladite forme pharmaceutique solide manque d'un agent qui exerce un pouvoir tampon à un pH entre 2 et 6.

15. Procédé selon l'une quelconque des revendications 1 à 14, dans lequel la forme pharmaceutique solide renferme de l'acétate de desmopressine dans une quantité comprise entre 20 et 600 µg par unité de ladite forme pharmaceutique solide.
